(19) Europäisches Patentamt
European Patent Office
Office européen des brevets

(11) Veröffentlichungsnummer: **0 173 222**
**B1**

(12) # EUROPÄISCHE PATENTSCHRIFT

(45) Veröffentlichungstag der Patentschrift: **09.03.88**

(51) Int. Cl.⁴: **C 07 C 17/12**, C 07 C 25/02 // C07D327/08

(21) Anmeldenummer: **85110419.0**

(22) Anmeldetag: **20.08.85**

(54) **Verfahren zur Kernchlorierung von Toluol.**

(30) Priorität: **31.08.84 DE 3432095**

(43) Veröffentlichungstag der Anmeldung:
**05.03.86 Patentblatt 86/10**

(45) Bekanntmachung des Hinweises auf die Patenterteilung:
**09.03.88 Patentblatt 88/10**

(84) Benannte Vertragsstaaten:
**BE CH DE FR GB IT LI NL**

(56) Entgegenhaltungen:
**EP - A - 0 063 384**
**DE - A - 3 023 437**
**JP - A - 56 110 630**
**US - A - 4 069 264**

(73) Patentinhaber: **HOECHST AKTIENGESELLSCHAFT, Postfach 80 03 20, D-6230 Frankfurt am Main 80 (DE)**

(72) Erfinder: **Wolfram, Hans, Dr., Johann-Strauss-Strasse 40, D-6233 Kelkheim (Taunus) (DE)**

**EP 0 173 222 B1**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

**Beschreibung**

Die Kernchlorierung von Toluol erfolgt bekanntlich nach den für die Kernhalogenierung von aromatischen Verbindungen üblichen Methoden. Bei der Monochlorierung des Toluols entstehen hauptsächlich o- und p-Chlortoluol neben einer untergeordneten Menge des m-Isomeren sowie gegebenenfalls auch von höher und in der Methylgruppe chlorierten Produkten. Von den kernchlorierten Monochlortoluolen besitzt insbesondere das p-Isomere wirtschaftliche Bedeutung als Zwischenprodukt für zahlreiche organische Synthesen. Es hat daher nicht an Versuchen gefehlt, die Kernchlorierung des Toluols in Richtung auf eine Erhöhung des p-Chlortoluol-Anteils hin zu lenken.

So soll z.B. nach dem in der JP-OS 56 (= 1981) — 110 630 der Firma Hodogaya Kagaku Kogyo K.K. beschriebenen Verfahren der Kernchlorierung von Toluol eine erhöhte Bildung des p-Cl-Isomeren dadurch erreicht werden, dass man — zusätzlich zu den üblichen Lewis-Säure-Katalysatoren — noch bestimmte (poly-)halogenierte Phenoxathiine als Cokatalysatoren zusetzt. Für die Cokatalysatoren ist folgende Formel angegeben:

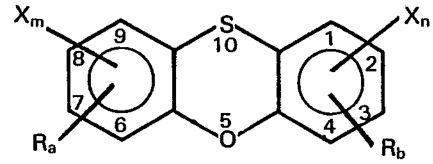

worin R = H oder $C_1$-$C_4$-Alkyl,
a = 0 bis 4-m,
b = 0 bis 4-n,
x = Cl, Br, F,
m + n = 2 bis 8.

Die Cokatalysatoren werden nach den Angaben der genannten JP-OS hergestellt durch Umsetzung von unsubstituiertem oder einem methylsubstituierten Diphenylether mit Schwefel oder einem Schwefelchlorid in Gegenwart von $AlCl_3$ als Katalysator entsprechend der Vorschrift in Organic Syntheses Coll. Vol. 2, S. 485/86, mit nachfolgender Halogenierung. Die Halogenierung erfolgt (nach den Beispielen der JP-OS) mit Chlor oder Brom in Gegenwart eines $SbCl_5$-Katalysators bei Temperaturen zwischen 70 und 180°C in $CCl_4$ als Lösungsmittel. Für die Herstellung beispielsweise des 4fach chlorierten 3-Methylphenoxathiins lässt sich folgendes Reaktionsschema angeben:

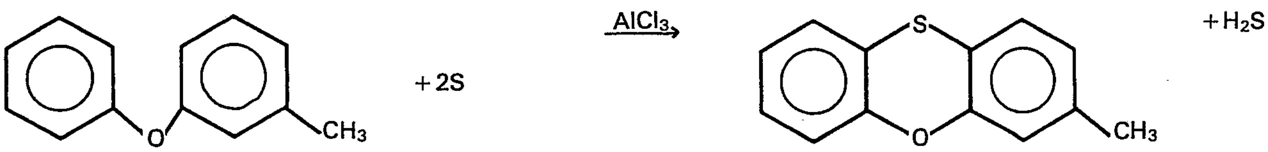


m-Methyl-diphenylether

3-Methyl-phenoxathiin

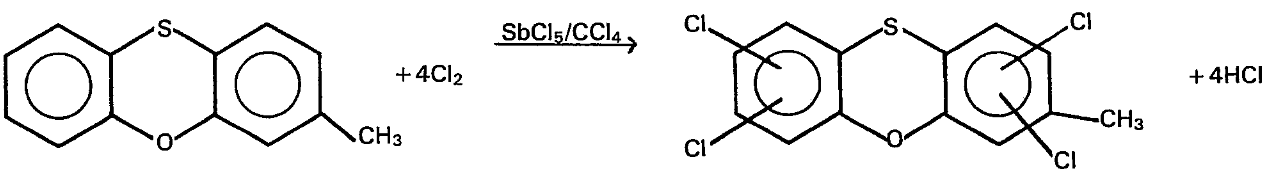


Die Chlorsubstituenten in der End-Verbindung können allerdings auch anders verteilt sein, d.h. es kann auch einer der beiden endständigen aromatischen Ringe 3- oder 4mal und der andere dafür nur einmal bzw. überhaupt nicht durch Chlor substituiert sein.

Das günstigste Ausführungsbeispiel in der JP-OS — d.h. das Beispiel, in welchem das höchste Verhältnis p-/o-Chlortoluol sowie auch das höchste Verhältnis p-/Monochlortoluol (o + m + p) erhalten wird — ist Beispiel Nr. 6. Das p-/o-Verhältnis beträgt dort 50,3/37,9 = 1,33, das Verhältnis p-/Monochlortoluol = 57%. Das Beispiel arbeitet mit $SbCl_3$ als Katalysator und mit chloriertem 3-Methylphenoxathiin des Chlorierungsgrads 4,8 als Cokatalysator bei 20°C.

Als bevorzugte halogenierte Phenoxathiine sind in der vorerwähnten JPOS u.a. auch chlorierte Dimethylphenoxathiine — und darunter auch Dimethyltetrachlorphenoxythiin — namentlich genannt. Über die Stellungen der Methylgruppen und der Chloratome sind jedoch keine Aussagen gemacht; auch Ausführungsbeispiele mit chlorierten Dimethylphenoxathiinen sind nicht vorhanden. Wegen der praktisch unüberschaubar grossen Zahl von denkbaren isomeren chlorierten Dimethylphenoxathiinen ist die diesbezügliche Erwähnung in der genannten JP-OS ohne konkrete Aussagekraft hinsichtlich irgendwelcher bestimmter Isomerer.

Die in der vorerwähnten JP-OS beschriebenen Phenoxathiin-Cokatalysatoren wurden von der Firma Hodogaya Kagaku Kogyo K.K. zwecks weiterer Erhöhung des p-Isomeren-Anteils bei der Kernchlorierung von Toluol in Gegenwart von Lewis-Säure-Katalysatoren weiterentwickelt. Nach der EP-OS 0 063 384 dieser Firma werden als Cokatalysatoren andere spezielle chlorierte Phenoxathiine verwendet. Vor der Chlorierung besitzen diese Phenoxathiine folgende Formel:

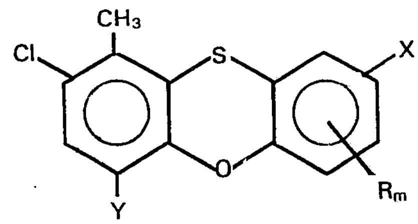

worin R = $CH_3$ in 1- und/oder 3-Stellung,
m = 0, 1 oder 2,
X = H oder Cl,
Y = Cl oder $CH_3$,
mit m = 1 oder 2 und X = H oder Cl für Y = Cl,
X = Cl für Y = $CH_3$ und m = 0, sowie
X = H für Y = $CH_3$ und m + 1 oder 2.

Für die Wirksamkeit als Cokatalysatoren soll (lt. S. 7 EP-OS) wesentlich sein, dass (vor der Chlorierung)

A) 9-Methyl-6,8-dichlorphenoxathiin in 1- und/oder 3-Stellung Methylgruppe(n) und in 2-Stellung ein H- oder Cl-Atom besitzt, oder

B) dass 6,9-Dimethyl-8-chlorphenoxathiin in 1- und/oder 3-Stellung durch Methyl, oder in 2-Stellung durch Cl substituiert ist, falls in 1- und/oder 3-Stellung keine Methylgruppe sitzt.

Die in dieser EP-OS genannten Phenoxathiine werden im Prinzip in der gleichen Weise hergestellt, wie dies auch in der vorerwähnten JP-OS für die dortigen Cokatalysatoren beschrieben ist. In der EP-OS ist allerdings auch noch angegeben, wie man zu den entsprechenden Ausgangs-Diphenylethern gelangt [durch Ullmann-Reaktion von Bromaromaten mit Phenol (-Derivaten) in Gegenwart eines Kupferkatalysators], z.B.

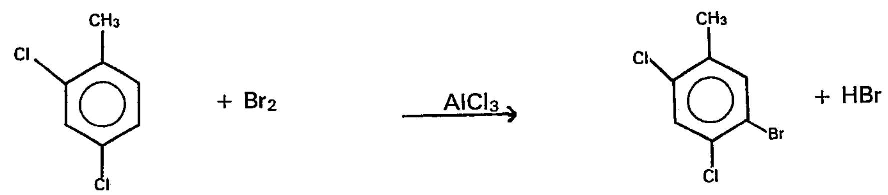


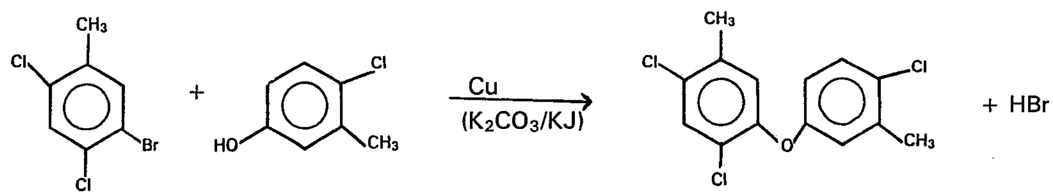


Bezüglich des Erhalts des höchsten Anteils an p-Chlortoluol sind die günstigsten Beispiele der EP-OS die Beispiele 15 und 16.

In Beispiel 15 ist das p-/o-Verhältnis 59,1/39,1 = 1,51, und der p-/Monochlortoluol-Anteil 60,4%. Das Beispiel arbeitet mit $SbCl_5$ als Katalysator und chloriertem 3,6,9-Trimethyl-8-chlor-phenoxathiin des Durchschnittschlorierungsgrads 3,1 als Cokatalysator bei 20°C (Einchlorierungsgrad 0,99).

In Beispiel 16 wird ein p-/o-Verhältnis von 56,8/36,8 = 1,54 und ein p-/Monochlortoluol-Anteil von 60,7% angegeben. Dieses Beispiel arbeitet mit $SbCl_3$ als Katalysator und chloriertem 3,6,9-Trimethyl-8-chlor-phenoxathiin des Chlorierungsgrads 2,0 als Cokatalysator, ebenfalls bei 20°C (Einchlorierungsgrad 0,94).

Weiterhin ist aus der DE-A-3 023 437, Beispiel 3, Nr. 19 und Beispiel 6, Tabelle 4, Nr. 1 bekannt, Toluol in Gegenwart von Antimontrichlorid und von 2,8-Dimethyl-3,7-dichlorphenoxathiin zu chlorieren. Dabei werden 2-Chlortoluol und 4-Chlortoluol im Verhältnis von 0,85 (Beispiel 3, Nr. 19) bzw. 0,85 bis 0,87 (Beispiel 6, Tabelle 4, Nr. 1) gebildet, was einem p-/o-Verhältnis von 1,18 bzw. einem Bereich von 1,15 bis 1,18 entspricht.

Obwohl die insbesondere nach der EP-A-0 063 384 erreichten p-/o- und p-Monochlortoluol-Verhältnisse nicht ungünstig sind, war es wegen des auch dabei noch entstehenden recht erheblichen Anteils vor allem an dem o-Isomeren wünschenswert und bestand die Aufgabe, die Kernchlorierung von Toluol weiter zugunsten der Bildung des p-Monochlortoluols zu verbessern, zumal in der vorerwähnten EP-OS auch ausgesprochen wird (vgl. Seite 2, Absatz 2), dass eine Erhöhung des p-/Monochlortoluol-Anteils schon um 0,5% von hohem wirtschaftlichen Wert ist.

Die Aufgabe konnte erfindungsgemäss durch den Einsatz von chloriertem 2,8-Dimethyl-phenoxathiin gelöst werden.

Erfindungsgegenstand ist daher ein Verfahren zur Kernchlorierung von Toluol in Gegenwart von Lewis-Säuren als Katalysatoren und von chloriertem Dimethyl-phenoxathiin als Cokatalysator, das dadurch gekennzeichnet ist, dass man als chloriertes Dimethylphenoxathiin das Produkt verwendet, welches erhalten wird durch Chlorierung von 2,8-Dimethyl-phenoxathiin mit etwa 4 Mol $Cl_2$/Mol 2,8-Dimethyl-phenoxathiin in Gegenwart einer Lewis-Säure — vorzugsweise von $SbCl_3$ und/oder $SbCl_5$ — als Katalysator bei Temperaturen zwischen etwa 70 und 120°C, gegebenenfalls in einem inerten Lösungsmittel, und welches hauptsächlich aus 1,3,7,9-Tetrachlor-2,8-dimethyl-phenoxathiin der Formel

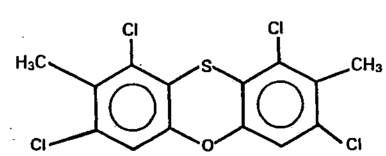

besteht.

Dadurch wird ein p-/o-Verhältnis von meist über 1,6 und ein p-/Monochlortoluol-Anteil von meist über 62% erreicht — was im Hinblick auf die vorerwähnte Aussage auf Seite 2, Absatz 1 der EP-OS 0 063 384 einen erheblichen Fortschritt bedeutet.

Die erfindungsgemäss geschaffene Möglichkeit der weiteren Verschiebung des Isomeren-Verhältnisses zugunsten des p-Isomeren bei der Kernchlorierung des Toluols ist sehr überraschend, weil aufgrund der Vielzahl der in der JP-OS 56-110 630 sowie der EP-OS 0 063 384 als Cokatalysatoren beschriebenen sehr ähnlichen Phenoxathiin-Derivate nicht zu erwarten war, dass ein nur relativ geringfügig abgewandeltes anderes Phenoxathiin-Derivat nochmals eine Verbesserung ergibt und weil das erfindungsgemäss eingesetzte Phenoxathiin-Derivat auch die Kriterien, welche in der EP-OS 0 063 384 (Seite 7) für die entsprechende cokatalytische Wirksamkeit angegeben sind, nicht erfüllt.

Der erfindungsgemäss eingesetzte Cokatalysator wird im Prinzip hergestellt, wie dies in der JP-OS 56-110 630 und der EP-OS 0 063 384 für die dort beschriebenen Phenoxathiin-Derivate angegeben ist. Im vorliegenden Fall geht man von Di-p-tolylether aus, der seinerseits z.B. durch Ullmann-Reaktion von p-Chlotoluol und p-Kresol erhältlich ist.

Der Di-p-tolylether wird dann mit Schwefel in Gegenwart von $AlCl_3$, erhitzt, wobei 2,8-Dimethyl-phenoxathiin entsteht:

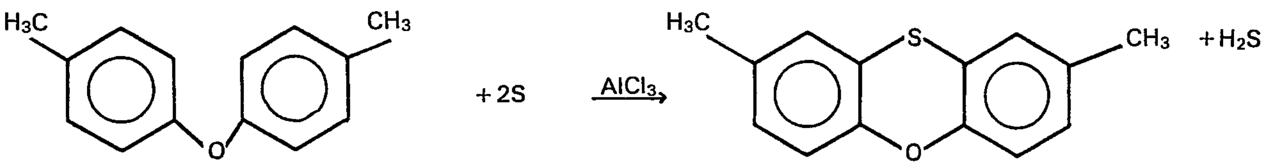


Die Chlorierung des 2,8-Dimethyl-phenoxathiins erfolgt mit etwa 4 Mol Chlor/Mol 2,8-Dimethyl-phenoxathiin in Gegenwart einer Lewis-Säure als Katalysator. Als Lewis-Säure-Katalysatoren kommen im Prinzip alle bekannten Lewis-Säuren in Frage, wie z.B. die Oxide und Halogenide von Al, Sn, Ti, Sb, Fe usw.; bevorzugt sind $SbCl_3$ und/oder $SbCl_5$.

Die Katalysatorkonzentration liegt im allgemeinen zwischen etwa 0,001 und 5 Gew.-%, vorzugsweise zwischen etwa 0,1 und 2 Gew.-%, bezogen auf das 2,8-Dimethyl-phenoxathiin.

Die Reaktionstemperatur für die Chlorierung liegt zwischen etwa 70 und 120°C.

Die Chlorierung kann sowohl in Abwesenheit als auch in Anwesenheit von inerten Lösungsmitteln durchgeführt werden; bevorzugt ist jedoch die Anwesenheit solcher Lösungsmittel, weil der Ansatz dadurch besser handhabbar ist. Als inerte Lösungsmittel kommen vorzugsweise niedere aliphatische Chlorkohlenwasserstoffe wie z.B. Tetrachlorkohlenstoff oder Tetrachlorethylen in Frage.

Die mit Hilfe des Lösungsmittels hergestellten Lösungen sind vorteilhaft etwa 5 bis 20 (Gew.-) %ig an 2,8-Dimethyl-phenoxathiin.

Bei der Chlorierung des 2,8-Dimethyl-phenoxathiins in der vorstehend beschriebenen Weise entsteht hauptsächlich — d.h. zu mindestens etwa 50% — 1,3,7,9-Tetrachlor-2,8-dimethyl-phenoxathiin. Als Nebenprodukte werden geringere Mengen vor allem von 3fach und von 5fach chloriertem 2,8-Dimethyl--phenoxathiin gebildet. Die Struktur der Verbindungen wurde mittels NMR-Spektroskopie sichergestellt.

Der Schwefelgehalt des Chlorierungsproduktes liegt normalerweise zwischen etwa 8 und 9%, der Chlorgehalt zwischen etwa 37 und 39%. Die theoretischen Werte für Tetrachlordimethyl-phenoxathiin liegen bei 8,74% (S) bzw. 38,7% (Cl).

Das erfindungsgemässe Verfahren zur Kernchlorierung von Toluol wird ansonsten in praktisch der gleichen Weise durchgeführt, wie dies für die entsprechenden Verfahren in der JP-OS 56-110 630 und der EP-OS 0 063 384 beschrieben ist — nur dass eben im vorliegenden Fall ein anderer Cokatalysator verwendet wird.

Der Einchlorierungsgrad beträgt höchstens = 1. Darüberliegende Werte würden verstärkt zu einer unerwünschten Mehrfachchlorierung führen.

Als Lewis-Säure-Katalysatoren können praktisch alle möglichen Lewis-Säuren verwendet werden; einige beispielhafte Lewis-Säuren sind vorstehend bei der Beschreibung der Chlorierung des 2,8-Dimethyl-phenoxathiins aufgezählt.

Die Menge des Katalysators sowie des Cokatalysators liegt normalerweise jeweils zwischen etwa 0,005 und 5 Gew.-%, vorzugsweise zwischen etwa 0,05 und 0,5 Gew.-%, bezogen auf das Ausgangs-Toluol.

Die Toluolchlorierung wird vorteilhaft im Temperaturbereich zwischen etwa 0 und 80°C, insbesondere zwischen etwa 0 und 40°C, durchgeführt.

Als Reaktionsdruck ist Normaldruck bevorzugt, wenngleich unter bestimmten Umständen auch Unter- und Überdruck möglich sind.

Zwecks Verdünnung des Reaktionsansatzes kann gegebenenfalls noch ein inertes Lösungsmittel zugesetzt werden; dies bringt jedoch keinen besonderen Vorteil.

Das Verfahren ist sowohl kontinuierlich als auch diskontinuierlich durchführbar.

Die Aufarbeitung des Reaktionsansatzes erfolgt auf übliche Weise, vorzugsweise destillativ. Wegen der geringeren o- und m-Chlortoluol-Anteile sind bei der destillativen Reindarstellung des p-Chlortoluols keine extrem hohen Trennleistungen mehr erforderlich.

Technisch von Bedeutung ist ferner, dass der Cokatalysator selbst aus dem Rohchlorierungsgemisch nach Abdestillieren der flüssigen Bestandteile

wiedergewonnen und erneut eingesetzt werden kann, ohne an Wirksamkeit zu verlieren.

Die folgenden Beispiele sollen der weiteren Erläuterung der Erfindung dienen. Vor den Erfindungsbeispielen wird die Darstellung des Cokatalysators wiedergegeben.

*Darstellung des Cokatalysators*

a) 2,8-Dimethyl-phenoxathiin

120 g (= 0,6 Mol) Di-p-tolylether wurden in einem Rührkolben aufgeschmolzen und mit 45 g (= 0,34 Mol) $AlCl_3$ versetzt; hierauf wurden bei 70 bis 80°C 20 g (= 0,625 Mol) Schwefel eingetragen. Es wurde auf 100°C erhitzt und einige Stunden bei dieser Temperatur gehalten. Die Mischung wurde dann durch Eingiessen in verdünnte Salzsäure zersetzt, worauf sich ein schweres Öl abschied, welches abgetrennt wurde. Das Öl wurde getrocknet und zur Isolierung des 2,8-Dimethyl-phenoxathiins im Vakuum fraktioniert.

Die Fraktionierung des Rohöls — ca. 127 g — lieferte 48% des eingesetzten Di-p-tolylethers als Vorlauf zurück. Nach einem geringfügigen Zwischenlauf destillierte das 2,8-Dimethyl-phenoxathiin mit einem Ep von > 68°C über.

Ausbeute: 60,5 g (= 44% d.Th., = 84% bezogen auf den umgesetzten Di-p-tolylether)

Der Schwefelgehalt betrug 14,3% (Th. 14,0%).

b) Chloriertes 2,8-Dimethyl-phenoxathiin

114 g (= 0,5 Mol) 2,8-Dimethyl-phenoxathiin wurden in 600 ml = 1000 g (= 6 Mol) Tetrachlorethylen gelöst und mit 2 g $SbCl_3$ versetzt.

Die Lösung wurde auf 100°C erwärmt. Dann wurden 140 g (= 2 Mol) Chlorgas innerhalb von 5 bis 6 Stunden eingeleitet. Darauf wurde auf ca. 60°C gekühlt und mit 800 ml Methanol versetzt, wobei das chlorierte Produkt kristallin ausfiel. Bei 30°C wurden die Kristalle abgenutscht, auf der Nutsche mit Methanol gewaschen, trockengesaugt und im Vakuum getrocknet. Fp. 145-150°C.

| | |
|---|---|
| Ausbeute: | 150 g = 82% d.Th. |
| Schwefelgehalt: | 9,0% (Th. 8,74% S für Tetrachlorverbindung) |
| Chlorgehalt: | 37,5% (Th. 38,7% Cl für Tetrachlorverbindung) |

*Erfindungsbeispiele:*

*Beispiel 1*

1400 g (= 15,2 Mol) Toluol wurden mit 2 g $SbCl_3$ sowie mit 4 g des — wie vorstehend beschrieben — hergestellten Cokatalysators versetzt. Unter Kühlung wurde bei 20°C ein mässiger Chlorstrom bis zu einer Dichte von 1,043 eingeleitet. Die intensiv blaugrün gefärbte Lösung wurde neutral gewaschen. Das Rohgemisch enthielt noch 16% Toluol.

Die Monochlortoluolfraktion setzte sich zusammen aus

37 % o-,
0,4% m- und
61,6% p-Chlortoluol.

An Rückstand verblieben ca. 3 bis 4 g.

Das p-/o-Verhältnis war somit 1,66, der p-/Monochlortoluol-Anteil 62,2%.

*Beispiel 2*

Beispiel 1 wurde wiederholt; es wurde jedoch bis zu einer Dichte des Gemisches von 1,064 chloriert. Die Aufarbeitung erfolgte wie in Beispiel 1. Das Rohgemisch enthielt noch 6,9% Toluol.

Die Monochlortoluolfraktion setzte sich zusammen aus

36 % o-,
0,3% m- und
60 % p-Chlortoluol.

Der Rückstand betrug 3 g (hauptsächlich Katalysator).

Das p-/o-Verhältnis war hier also 1,67, der p-Monochlortoluol-Anteil 62,3%.

*Beispiel 3*

Es wurde wiederum in gleicher Weise wie in Beispiel 1 gearbeitet, nur dass anstelle des $SbCl_3$ als Lewis-Säure-Katalysator jetzt 1 g $FeCl_3$ eingesetzt und die Chlorierung bei 5°C durchgeführt wurde. Es wurde — wie in Beispiel 1 — bis zu einer Dichte des Gemisches von 1,043 chloriert. Nach dem Neutralwaschen der intensiv blaugrün gefärbten Lösung erhielt das Rohgemisch noch 15% Toluol.

Die Monochlortoluolfraktion setzte sich zusammen aus

1,1% Toluol,
40 % 0-,
0,3% m- und
59 % p-Chlortoluol.

Das p-/o-Verhältnis war hier 1,475 und der p-Monochlortoluol-Anteil 60%.

*Beispiel 4*

Es wurde Toluol mit dem aus Beispiel 1 zurückgewonnenen Cokatalysator chloriert.

Die Rückgewinnung des Cokatalysators aus Beispiel 1 folgte durch Aufnahme des fast trockenen Rückstandes, welcher bei der Fraktionierung des Chlorierungsgemisches nach Beispiel 1 anfiel mit Methanol, Abfiltration und Trocknung. Es wurden ca. 3 g mit einem Fp. von 148-159°C erhalten.

Zur Chlorierung wurde 2 g des zurückgewonnenen Cokatalysators in 700 g (= 7,6 Mol) Toluol gelöst und mit 1 g $SbCl_3$ versetzt und bei 20°C bis zu einer Dichte von 1,045 mit Chlorgas umgesetzt.

Die Monochlortoluolfraktion enthielt 59% p-Chlortoluol. Der Kolbeninhalt wurde dabei bis zur Trockne abdestilliert. Die ungeschmälerte Wirksamkeit des zurückgewonnenen Cokatalysators ist damit augenfällig.

**Patentansprüche**

1. Verfahren zur Kernchlorierung von Toluol in Gegenwart von Lewis-Säuren als Katalysatoren und von chloriertem Dimethyl-phenoxathiin als Cokatalysator, dadurch gekennzeichnet, dass man als chloriertes Dimethyl-phenoxathiin das Produkt verwendet, welches erhalten wird durch Chlorierung von

2,8-Dimethyl-phenoxathiin mit etwa 4 Mol Chlor/Mol 2,8-Dimethyl-phenoxathiin in Gegenwart einer Lewis-Säure — vorzugsweise von $SbCl_3$ und/oder $SbCl_5$ — als Katalysator bei Temperaturen zwischen etwa 70 und 120°C, gegebenenfalls in einem inerten Lösungsmittel, und welches hauptsächlich aus 1,3,7,9-Tetrachlor-2,8-dimethyl-phenoxathiin der Formel

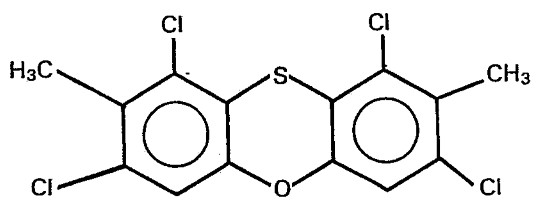

besteht.

2. Verfahren nach Anspruch 1, dadurch gekennzeichnet, dass das chlorierte 2,8-Dimethyl-phenoxathiin einen Schwefelgehalt von etwa 8 bis 9% und einen Chlorgehalt von etwa 37 bis 39% besitzt.

3. Verfahren nach den Ansprüchen 1 oder 2, dadurch gekennzeichnet, dass man die Kernchlorierung des Toluols bis zu einem Einchlorierungsgrad von höchstens 1 führt.

4. Verfahren nach einem oder mehreren der Ansprüche 1 bis 3, dadurch gekennzeichnet, dass man den Katalysator und den Cokatalysator in einer Menge von jeweils etwa 0,005 bis 5 Gew.-%, vorzugsweise jeweils etwa 0,05 bis 0,5 Gew.-%, bezogen auf das Ausgangs-Toluol, verwendet.

5. Verfahren nach einem oder mehreren der Ansprüche 1 bis 4, dadurch gekennzeichnet, dass man die Kernchlorierung bei Temperaturen zwischen etwa 0 und 80°C, vorzugsweise zwischen etwa 0 und 40°C, durchführt.

**Claims**

1. A process for ring-chlorinating toluene in the presence of Lewis acids as catalysts and of chlorinated dimethylphenoxathiin as cocatalyst, characterized in using as the chlorinated dimethylphenoxathiin the product which is obtained by chlorination of 2,8-dimethylphenoxathiin with about 4 mol of chlorine/mol of 2,8-dimethylphenoxathiin in the presence of a Lewis acid — preferably $SbCl_3$ and/or $SbCl_5$ — as catalyst at temperatures between about 70 and 120°C, if desired in an inert solvent, and which comprises in the main 1,3,7,9-tetrachloro-2,8-dimethylphenoxathiin of the formula

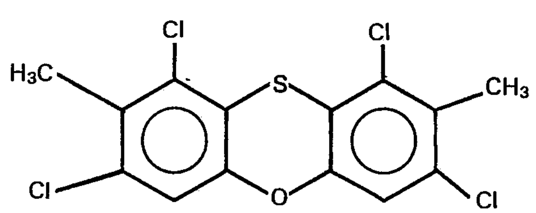

2. The process as claimed in claim 1, wherein the chlorinated 2,8-dimethylphenoxathiin has a sulfur content of about 8 to 9% and a chlorine content of about 37 to 39%.

3. The process as claimed in claim 1 or 2, wherein the ring chlorination of toluene leads to a chlorination degree of at most 1.

4. The process as claimed in one or more of claims 1 to 3, wherein the catalyst and the cocatalyst are each used in an amount of about 0.005 to 5% by weight, preferably about 0.05 to 0.5% by weight, relative to starting toluene.

5. The process as claimed in one or more of claims 1 to 4, wherein the ring chlorination is carried out at temperatures between about 0 and 80°C, preferably between about 0 and 40°C.

**Revendications**

1. Procédé pour chlorer le toluène sur son noyau, en présence d'acides de Lewis comme catalyseurs et d'une diméthyl-phénoxathiinne chlorée comme cocatalyseur, procédé caractérisé en ce qu'on utilise, comme diméthyl-phénoxathiinne chlorée, le produit que l'on obtient en chlorant la diméthyl-2,8 phénoxathiinne avec environ 4 mol de chlore par mole de diméthyl-2,8 phénoxathiinne, en présence d'un acide de Lewis, de préférence en présence de $SbCl_3$ et/ou de $SbCl_5$, comme catalyseur, à des températures comprises entre environ 70 et 120°C, éventuellement dans un solvant inerte, et qui est principalement constitué par la tétrachloro-1,3,7,9 diméthyl-2,8 phénoxathiinne, composé qui répond à la formule suivante:

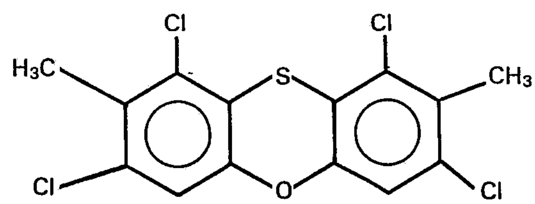

2. Procédé selon la revendication 1, caractérisé en ce que la diméthyl-2,8 phénoxathiinne chlorée a une teneur en soufre d'environ 8 à 9% et une teneur en chlore d'environ 37 à 39%.

3. Procédé selon l'une des revendications 1 et 2, caractérisé en ce que la chloration du toluène sur son noyau est effectuée jusqu'à un taux de monochloration d'au plus 1.

4. Procédé selon une ou plusieurs des revendications 1 à 3, procédé caractérisé en ce qu'on utilise le catalyseur et le co-catalyseur chacun en une quantité d'environ 0,005 à 5% en poids, de préférence d'environ 0,05 à 0,5% en poids, par rapport au toluène de départ.

5. Procédé selon une ou plusieurs des revendications 1 à 4, caractérisé en ce qu'on effectue la chloration sur le noyau à des températures comprises entre environ 0 et 80°C, de préférence entre environ 0 et 40°C.